# EUROPEAN PATENT SPECIFICATION

(11) **EP 2 133 103 B1**
(45) Date of publication and mention of the grant of the patent: **19.10.2011**
(21) Application number: 08750387.6
(22) Date of filing: 13.03.2008
(51) Int. Cl.: A61L 9/015, A61L 9/14, H02J 3/14, G05F 3/02, H01R 13/66

(54) **PROGRAMMABLE ELECTRICAL SEQUENCER**
PROGRAMMIERBARER ELEKTRISCHER SEQUENZER
SÉQUENCEUR ÉLECTRIQUE PROGRAMMABLE

(30) Priority: 13.03.2007 ES 200700751
(43) Date of publication of application: 16.12.2009
(73) Proprietor: Francisco Aragon S.L., Molina de Segura Murcia (ES)
(72) Inventor: BLASCO FEO, Vicente, E-46003-Valencia (ES)
(74) Representative: Soler Lerma, Santiago
(86) International application number: PCT/ES2008/000144
(87) International publication number: WO 2008/110649

(56) References cited:
- EP-A2- 0 238 983
- EP-A2- 0 267 697
- WO-A1-2006/126139
- WO-A1-2007/064188
- AU-A4- 2005 101 001
- ES-A1- 0 413 076
- US-A1- 2004 037 764
- US-A1- 2005 012 401
- US-A1- 2006 193 611

## Description

This invention consists in a programmable electronic sequencer with a current collector and different current outlets such that the sequencer program determines which of the possible outlets the current will be directed to, as well as the intensity of this current and the time the current will be supplied for.

The sequencer proposed is of application in several fields, and one of these with undeniable advantages is that of home air fresheners, since several different devices can be connected to the device, such as vaporisers, where the sequencer's program will determine which of such devices will act at each moment, with what intensity and for how long. If at least one of these devices is an air freshener, this allows not only alternating between different fragrances, but also combining them or even balancing them out.

The technical field this invention belongs to is that of electronic sequencers, especially those intended to control air freshener devices.

### BACKGROUND OF THE INVENTION

The use of electric air fresheners that facilitate the volatilisation of essences by the heat generated by resistances is relatively frequent. These essences can be either liquid essences that come in a bottle with a soaked wick that is heated on its free end or solid essences in tablet form.

There is plenty of patent literature regarding ways of volatilising fragrances, either by sublimation or evaporation, most of which are in the public domain.

One of the main problems when attempting the long-lasting scenting of a room or atmosphere is the saturation of the pituitary membrane, which causes the fragrance to no longer be perceived by the user after a relatively short time.

Several solutions have been put forth to solve this problem, such as, therefore, Japanese patent JP11000391, which proposes a device that evaporates the essence by heating the free end of a soaked wick, incorporating a timer that alternates time periods with electrical current and therefore evaporation of the essence with time periods without electrical current that allow the pituitary membrane to become unaccustomed to the essence, and when the vaporiser is activated again the user once again perceives the fragrance.

There is also the possibility of the alternating regular supply of two aromatic compositions, as disclosed in patent ES2218183 by Reckitt Benckiser.

Two recent patents have also been published, WO2004/093928 by The Procter & Gamble Company, which suggests alternating two or more volatile compositions in a defined series of sequences, or AU2005101001 by Sara Lee, which also proposes alternating at least two fragrances.

All of these patents base their systems to prevent the pituitary membrane from getting used to the essence on sequentially activating or deactivating a series of fragrances, either in the case of Japanese patent JP11000391, where the device is activated or deactivated, or the case of patent ES2218183, which alternates two fragrances, or more recently patents WO2004/093928 or AU2005101001, which contemplate the alternating activation of two or more fragrances.

However, these patents do not contemplate the possibility of combining two or more fragrances in a controlled manner, that is, by having two or more active vaporisers, the intensity of evaporation of each of which can be varied and programmed independently.

The fact is that, due to the thermal inertia of the vaporisers, the transition between one fragrance and the next is not radical but gradual, and residues of the deactivated fragrance remaining in the atmosphere may combine with the first effects of the alternating fragrance.

However, this overlapping of the two fragrances is neither lasting nor controlled.

On the other hand, patent US2006/0193611 A1 suggests the configuration of a multi-fragrance air freshener to obtain a pleasant fragrance avoiding inadequate combinations of scents. To do this it uses compatible fragrances that have been pre-established by the manufacturer, but this only allows the connection of pre-established fragrance volatilisers to the device. It does not allow having an air freshening device that can combine different independent functions and produce a synergic effect between some of them, such as for example the combination of a fragrance and a fan that favours its fast and homogeneous diffusion, or an insect-attracting light together with an insecticide substance that enhances the action of such insecticide by gathering the insects around the device, thus not requiring great diffusion of the insecticide and avoiding damage to other living beings sharing the room such as children or pets.

In practice there are many and very diverse designs of electronic devices for several applications. Patent ES 413076A1 describes a device for the distribution of energy, the object of which is to achieve an average consumption of electricity that is much lower than the maximum consumption. However, the object of the device object of this invention is rather the contrary, that is, to generate the broadest possible differences in consumption, with peaks and valleys, in the different lines, such that, for example, in its use as an air freshener, the pituitary membrane does not become saturated, or in its combined use with an insecticide, for example, there will be peaks of consumption where the light will be greater and therefore there will be more insect attraction, and the need for insecticide vaporisation will be greater.

The invention proposed allows not only the use of the device for air freshening, keeping two or more vaporisers active and independently varying their intensity of operation as well as their activation time, therefore generating a balance of fragrances and fragrance combinations in a controlled manner, where it is the user who chooses the fragrances, but it also allows many other options according to the devices connected to each one of the outlets by the user.

### DESCRIPTION OF THE INVENTION

The invention is defined by the appended claims, the programmable electric sequencer having a current inlet and several outlets, as in the case of electrical distributors commonly known as multiplug adaptors, characterised in this case in that the sequencer itself is programmed to determine which of the current outlets the current is supplied to, for how long and with what intensity.

Each of the current outlets on this sequencer can be connected to different devices the activation time and operation intensity of which will be controlled by the sequencer, which will also determine whether each device is activated alone or together with other devices.

That is, each device connected to each outlet on the sequencer will have its own activation time and operation intensity, and two or more devices may be active at the same time.

In order to achieve this operation, the sequencer integrates a microprocessor that determines at each time the number of current lines active and the current intensity of each line, based on a program.
Since there can be different programming options, a selector has been provided for the user to be able to choose amongst the different programs.

The microprocessor described uses a series of thyristors, one for each current outlet line, to control the intensity of each current outlet line.

The microprocessor therefore determines which of the outlet lines it will supply current to, for how long and with what intensity, using the thyristors to control the intensity.

The devices connected to each of the sequencer's outlet lines will thus receive an electric current in a programmed manner, with the activation time and intensity determined by the sequencer.

In its use with fragrance vaporisers the combination possibilities for three fragrances, for example, are enormous, since it does not only allow the programming of an ordered alternation of each fragrance, but also all possible combinations of the activation of one, two, or all three fragrances with different combinations of the intensities of each fragrance and the duration for each one.

As explained above, there are many possibilities for combination preventing the pituitary membrane from becoming saturated, and one of the fragrances could therefore be replaced by another device, such as, for example, an insecticide or odour absorber, in which case the programming could activate these only when necessary.
There is no reason why other kinds of devices could not also be connected in order to provide specific functionality, such as for example a fan.

That is, by means of the present invention we have an air freshening device that can combine different independent functions and produce a synergic effect between some of them.

Thus, for example, if we place a fan on one of the current outlets and fragrances in the others, this makes diffusion of the fragrance in the place to be scented more homogeneous and faster.

Another possible example would be the following combination: one of the current outlets could be used with a light that attracts insects, and on another outlet or outlets there could be an insecticide substance, such that the insects concentrate around the device attracted by the light, resulting in a series of obvious advantages, since on the one hand the insects are gathered in a much smaller area and therefore the insecticide needs a much smaller radius of action than when not using an insect-attracting light, which leads to the use of less biocidal chemical substances and less inhaling of said substances by the user, all this still allowing the connection of an air freshener to another one of the current outlets.

Therefore, using the device of the present invention, we cannot only vaporise multi-fragrance substances, but we can also use synergic devices such as insecticides, fans or light, amongst others.

The programming of the microprocessor will determine in these cases the ideal activation periods for such devices.

The invention as proposed can incorporate a series of diodes at the ends of its lines that reduce consumption and allow the use of devices with small resistances by reducing the negative cycle of the alternating current, with obvious advantages.

### BRIEF DESCRIPTION OF THE DRAWINGS

In order to aid towards a better understanding of the invention, Figures are enclosed that are described below.

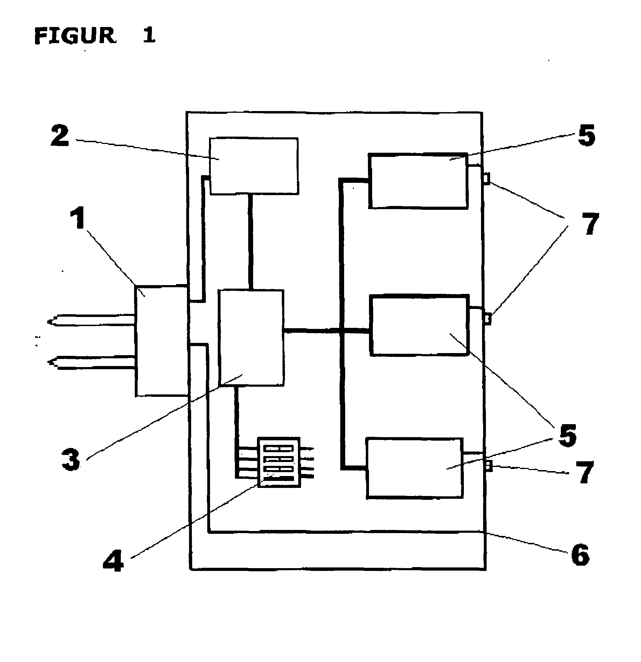

Figure 1 shows very schematically the internal distribution of the sequencer, where we can see the current inlet (1), the power source (2), the microprocessor (3), the program selector (4), the thyristors (5) on the current outlet lines controlled by the microprocessor (3) and a parallel current line direct from the power inlet (6).

The diagram shown in this Figure also shows the sensors operating on the outlet lines (7) to detect the presence or absence or a device connected to said line, such that if there are no devices connected to the line it will not be activated.

### DESCRIPTION OF AN EMBODIMENT OF THE INVENTION

There are different possible embodiments of the invention, since there can be variations in the number of outlets on the sequencer, the programming of the microprocessor, components can be added or removed from the electric circuit or it can be modified without such modifications being considered an independent invention from the one described here.

Of the different possible embodiments, we shall choose a sequencer with a single current inlet (1) and distributing to four different lines, of which three are subjected to the program of a microprocessor (3) and the fourth (6) is just an extension of the power inlet.

This fourth line (6) acts as a standard plug, such that the plug the sequencer is connected to is not limited only to service the sequencer, but can also be used for other appliances or devices, the plug for this fourth line therefore being a standard plug socket.

The other three current lines coming from the microprocessor will be controlled by its program, such that their activity or inactivity and the intensity of their current will depend on the program on the microprocessor (3).

In order to control the intensity of the current sent to the outer terminals of the sequencer, there are thyristors (5) installed on each line between the microprocessor and said terminals that will regulate the current intensity to that established by the microprocessor.

This microprocessor has different programs that allow the user to choose the one most suited to his or her needs or taste by means of a selector (4).

The programming possibilities are many and it is technically not complicated to add additional programs combining or alternating current lines or fragrance intensities.

The possibility of a program generating random operation such that it activates and deactivates the current lines and determines their intensity without a specific sequence is also contemplated.

Finally, the invention includes a sensor (7) at each outlet current line to determine whether there is a device connected to it or not, and if not, to prevent said line from being activated.

## Claims

1. A programmable electric sequencer for the combination and balancing of fragrances, comprising a current inlet (1) and several current outlets, **characterised in that** such current outlets being controlled by the action of a microprocessor (3) that determines at each moment the number of current outlets that are activated as well as the intensity of current in each outlet by means of a series of thyristors (5) arranged in line on each outlet, as well as sensors (7) that detect the presence or absence of devices connected to said current outlets.

2. A programmable electric sequencer and its application for the combination and balancing of fragrances according to the previous claim, **characterised in that** devices for fragrance vaporising can be installed at its current outlets so that the evaporation of the fragrance is activated or deactivated and varies in intensity according to the activation or deactivation and the intensity of the current line they are connected to all of it controlled by the action of a microprocessor

3. A programmable electric sequencer and its application for the combination and balancing of fragrances according to the previous claims, **characterised in that** two or more current lines can be activated at the same time.

4. A programmable electric sequencer and its application for the combination and balancing of fragrances according to previous claims, **characterised in that** the activity or inactivity, the duration and the intensity of action of each one of the outlet current lines is independent from the activity or inactivity, the duration or the intensity of the remaining outlet current lines.

5. A programmable electric sequencer and its application for the combination and balancing of fragrances according to previous claims, **characterised in that** each outlet current line being independent, these can be coordinated with one another by means of the programming of the microprocessor (3) to carry out combinations of the fragrances from the vaporisers.

6. A programmable electric sequencer and its application for the combination and balancing of fragrances according to previous claims, **characterised in that** each outlet current line being independent, these can be coordinated with one another by means of the programming of the microprocessor (3) to carry out the balancing of the fragrances from the vaporisers.

7. A programmable electric sequencer
for the combination and balancing of fragrances according to previous claims, **characterised in that** a supplementary device such as an insecticide or odour absorber is installed on one of the outlet current lines.

8. A programmable electric sequencer for the combination and balancing of fragrances according to previous claims, **characterised in that** there is a sensor (7) installed on each outlet current line to determine whether or not there is a device connected to it.

9. A programmable electric sequencer for the combination and balancing of fragrances according to previous claims, **characterised in that** only the outlet current lines where a device for fragrance vaporising is installed will be activated.

10. A programmable electric sequencer for the combination and balancing of fragrances according to the previous claims, **characterised in that** includes one or more current lines (6) that are not controlled by the microprocessor.

11. A programmable electric sequencer for the combination and balancing of fragrances according to the previous claims, **characterised in that** it includes a program selector (4).

## Patentansprüche

1. Programmierbarer elektrischer Sequenzer zur Kombination und Abstimmung von Duftstoffen, der einen Stromeingang (1) und mehrere Stromausgänge umfasst, **dadurch** ausgezeichnet, dass diese Stromausgänge von einem Mikroprozessor (3) gesteuert werden, der zu jedem Zeitpunkt sowohl die Anzahl der aktiven Stromausgänge als auch die Stromstärke an jedem Auslass mittels einer Reihe von Thyristoren (5) bestimmt, die an allen Ausgängen in Reihe geschaltet sind, wobei auch Sensoren (7) vorhanden sind, die das Vorhandensein von angeschlossenen Geräten an den besagten Stromausgängen feststellen.

2. Programmierbarer elektrischer Sequenzer und seine Anwendung zur Kombination und Abstimmung von Duftstoffen nach Anspruch 1, **dadurch gekennzeichnet, dass** sich an seinen Stromausgängen Geräte zur Zerstäubung von Duftstoffen installieren lassen, wodurch abhängig von der Aktivierung, Deaktivierung und Stärke der Ströme an den Ausgängen, an welchen diese Geräte angeschlossen sind, die Verdunstung der Duftstoffe aktiviert, deaktiviert oder in ihrer Intensität verändert wird, wobei diese Vorgänge von einem Mikroprozessor gesteuert sind.

3. Programmierbarer elektrischer Sequenzer und seine Anwendung zur Kombination und Abstimmung von Duftstoffen nach den vorherigen Ansprüchen, **dadurch gekennzeichnet, dass** sich zwei oder mehr Stromanschlüsse gleichzeitig aktivieren lassen.

4. Programmierbarer elektrischer Sequenzer und seine Anwendung zur Kombination und Abstimmung von Duftstoffen nach den vorherigen Ansprüchen, **dadurch gekennzeichnet, dass** Aktivität oder Inaktivität, Intensität und Aktivitätsdauer der einzelnen Stromausgänge voneinander unabhängig sind.

5. Programmierbarer elektrischer Sequenzer und seine Anwendung zur Kombination und Abstimmung von Duftstoffen nach den vorherigen Ansprüchen, **dadurch gekennzeichnet, dass** die einzelnen Stromausgänge voneinander unabhängig sind, wobei sie sich durch Programmierung des Mikroprozessors (3) miteinander koordinieren lassen, um Kombinationen der Duftstoffe aus den Zerstäubern zu erzeugen.

6. Programmierbarer elektrischer Sequenzer und seine Anwendung zur Kombination und Abstimmung von Duftstoffen nach den vorherigen Ansprüchen, **dadurch gekennzeichnet, dass** die einzelnen Stromausgänge voneinander unabhängig sind, wobei sie sich durch Programmierung des Mikroprozessors (3) miteinander koordinieren lassen, um die Duftstoffe aus den Zerstäubern untereinander abzustimmen.

7. Programmierbarer elektrischer Sequenzer für die Kombination und Abstimmung von Duftstoffen nach den vorherigen Ansprüchen, **dadurch gekennzeichnet, dass** an einem der Stromausgänge ein Zusatzgerät installiert ist, wie zum Beispiel eine Absorptionsvorrichtung für Insektizide oder Gerüche.

8. Programmierbarer elektrischer Sequenzer für die Kombination und Abstimmung von Duftstoffen nach den vorherigen Ansprüchen, **dadurch gekennzeichnet, dass** an jedem Stromausgang ein Sensor (7) installiert ist, der feststellt, ob dort ein Gerät angeschlossen ist.

9. Programmierbarer elektrischer Sequenzer für die Kombination und Abstimmung von Duftstoffen nach den vorherigen Ansprüchen, **dadurch gekennzeichnet, dass** nur jene Stromausgänge aktiviert werden, an welchen ein Gerät zur Zerstäubung von Duftstoffen installiert ist.

10. Programmierbarer elektrischer Sequenzer für die Kombination und Abstimmung von Duftstoffen nach den vorherigen Ansprüchen, **dadurch gekennzeichnet, dass** er einen Stromausgang oder mehrere Stromausgänge (6) umfasst, die nicht vom Mikroprozessor gesteuert werden.

11. Programmierbarer elektrischer Sequenzer für die Kombination und Abstimmung von Duftstoffen nach den vorherigen Ansprüchen, **dadurch gekennzeichnet, dass** er eine Programmwahlvorrichtung (4) umfasst.

## Revendications

1. Séquenceur électrique programmable pour la combinaison et l'équilibrage de fragrances, comprenant une entrée de courant (1) et plusieurs sorties de courant, **caractérisé en ce que** lesdites sorties de courant sont commandées par l'action d'un microprocesseur (3) qui détermine à chaque instant le nombre de sorties de courant qui sont activées ainsi que l'intensité du courant à chaque sortie au moyen d'une série de thyristors (5) disposés en ligne à chaque sortie, ainsi que des capteurs (7) qui détectent la présence ou l'absence de dispositifs reliés auxdites sorties de courant.

2. Séquenceur électrique programmable et son application pour la combinaison et l'équilibrage de fragrances selon la revendication précédente, **caractérisé en ce que** les dispositifs pour la vaporisation de fragrance peuvent être installés à leur sortie de courant de manière que l'évaporation de la fragrance est activée ou désactivée et varie en intensité selon l'activation ou désactivation et l'intensité de la ligne de courant à laquelle ils sont reliés, le tout étant commandé par l'action d'un microprocesseur

3. Séquenceur électrique programmable et son application pour la combinaison et l'équilibrage de fragrances selon les revendications précédentes, **caractérisé en ce que** deux ou plusieurs lignes de courant peuvent être activées au même instant.

4. Séquenceur électrique programmable et son application pour la combinaison et l'équilibrage de fragrances selon les revendications précédentes, **caractérisé en ce que** l'activité ou l'inactivité, la durée et l'intensité d'action de chacune des lignes de courant de sortie sont indépendantes de l'activité ou l'inactivité, la durée ou l'intensité du reste des lignes de courant de sortie.

5. Séquenceur électrique programmable et son application pour la combinaison et l'équilibrage de fragrances selon les revendications précédentes, **caractérisé en ce que** chaque ligne de courant de sortie étant indépendante, elles peuvent être coordonnées les unes aux autres au moyen de la programmation du microprocesseur (3) pour effectuer des combinaisons des fragrances des vaporisateurs.

6. Séquenceur électrique programmable et son application pour la combinaison et l'équilibrage de fragrances selon les revendications précédentes, **caractérisé en ce que** chaque ligne de courant de sortie étant indépendante, elles peuvent être coordonnées les unes aux autres au moyen de la programmation du microprocesseur (3) pour effectuer l'équilibrage des fragrances des vaporisateurs.

7. Séquenceur électrique programmable pour la combinaison et l'équilibrage de fragrances selon les revendications précédentes, **caractérisé en ce qu'**un dispositif supplémentaire tel qu'un insecticide ou absorbeur d'odeur est installé sur une des lignes de courant de sortie.

8. Séquenceur électrique programmable pour la combinaison et l'équilibrage de fragrances selon les revendications précédentes, **caractérisé en ce qu'**il y a un capteur (7) installé sur chaque ligne de courant de sortie pour déterminer s'il y a ou pas un dispositif relié à celle-ci.

9. Séquenceur électrique programmable pour la combinaison et l'équilibrage de fragrances selon les revendications précédentes, **caractérisé en ce que** seules les lignes de courant de sortie, où est installé un dispositif pour la vaporisation de fragrance, seront activées.

10. Séquenceur électrique programmable pour la combinaison et l'équilibrage de fragrances selon les revendications précédentes, **caractérisé en ce qu'**il comporte une ou plusieurs lignes de courant (6) qui ne sont pas commandées par le microprocesseur.

11. Séquenceur électrique programmable pour la combinaison et l'équilibrage de fragrances selon les revendications précédentes, **caractérisé en ce qu'**il comporte un sélecteur de programme (4).
